# EUROPÄISCHE PATENTSCHRIFT

(11) **EP 3 180 608 B1**
(45) Veröffentlichungstag und Bekanntmachung des Hinweises auf die Patenterteilung: **23.05.2018**
(21) Anmeldenummer: 15741158.8
(22) Anmeldetag: 20.07.2015
(51) Int. Cl.: G01N 31/12

(54) **ANALYSEGERÄT ZUR BESTIMMUNG EINER VON DER KONZENTRATION EINES ODER MEHRERER INHALTSSTOFFE EINER PROBE ABHÄNGIGEN MESSGRÖSSE**
ANALYSIS DEVICE FOR DETERMINING A MEASUREMENT QUANTITY THAT DEPENDS ON THE CONCENTRATION OF ONE OR MORE CONSTITUENTS OF A SAMPLE
APPAREIL D'ANALYSE POUR LA DÉTERMINATION D'UNE GRANDEUR DE MESURE DÉPENDANT DE LA CONCENTRATION D'UN OU PLUSIEURS COMPOSANTS D'UN ÉCHANTILLON

(30) Priorität: 12.08.2014 DE 102014111506
(43) Veröffentlichungstag der Anmeldung: 21.06.2017
(73) Patentinhaber: Analytik Jena AG, 07745 Jena (DE)
(72) Erfinder: EHRLING, Christiane, 98693 Ilmenau (DE); HENNEBERG, Heiko, 99338 Plaue (DE)
(74) Vertreter: Andres, Angelika Maria
(86) Internationale Anmeldenummer: PCT/EP2015/066507
(87) Internationale Veröffentlichungsnummer: WO 2016/023707

(56) Entgegenhaltungen:
- DE-A1- 19 826 496
- DE-T3- 69 801 053
- DE-U1-202012 102 724

## Beschreibung

Die Erfindung betrifft ein Analysegerät zur Bestimmung einer von der Konzentration eines oder mehrerer, insbesondere oxidierbarer, Inhaltsstoffe einer Probe abhängigen Messgröße.

Eine solche Messgröße kann beispielsweise eine Konzentration einer oder mehrerer Substanzen oder eines Elements, z.B. Schwefel, Chlor oder Wasserstoff, in der Probe oder ein Summenparameter wie der gesamte organisch gebundene Kohlenstoffgehalt (Total Organic Carbon, Abk. TOC), ein Gesamtgehalt an Kohlenstoff (organisch und anorganisch gebunden, Abk. TC) oder der gesamte gebundene Stickstoff (Total Nitrogen, Abk. TN_{b}), sein.

Bekannte Analysegeräte zur automatisierten Bestimmung solcher Messgrößen umfassen einen Aufschlussreaktor, in den eine feste, flüssige oder gasförmige Probe eindosiert wird. In dem Aufschlussreaktor, der beispielsweise als Pyrolyserohr ausgestaltet sein kann, werden die Inhaltsstoffe der Probe thermisch aufgeschlossen. Dabei wird organisch und anorganisch gebundener Kohlenstoff zu Kohlendioxid CO₂, Stickstoff zu Stickstoffoxid NOₓ, Schwefel zu Schwefeloxiden SO₂/SO₃, Chlor zu Chlorwasserstoff HCl und Wasserstoff zu Wasser H₂O umgesetzt. Das dabei entstehende Gas bzw. Gasgemisch wird mit Hilfe eines permanent den Aufschlussreaktor durchströmenden Trägergases, das in der Regel auch den nötigen Reaktionssauerstoff liefert, durch eine Trocknungs- und Absorbereinheit einer Messeinrichtung zugeführt, die zur Bestimmung der Messgröße dient. In einigen Anwendungen, insbesondere für die Analytik im Spurenbereich, wird als Reaktions- und Trägergas hochreiner Sauerstoff verwendet. Die Messeinrichtung umfasst ein oder mehrere spezifische Detektoren, die dazu dienen, den Anteil der für die zu bestimmende Messgröße relevanten Oxidationsprodukte in dem dem Detektor zugeleiteten Gasstrom zu ermitteln. Soll beispielsweise der TOC-Wert der Probe ermittelt werden, dient als spezifischer Detektor ein Infrarot-Detektor, der den CO₂-Gehalt des Gasstroms bestimmt, aus dem ein Messwert des TOC-Werts der Probe abgeleitet werden kann. Zur TN_{b}-Bestimmung kann ein NOₓ-Gehalt des Gasstroms mittels einer Chemilumineszenz-Messung bestimmt werden.

Die Bereitstellung von Sauerstoff als Trägergas bzw. als Reaktionspartner für den thermischen Aufschluss der Proben erfolgt in herkömmlichen Analysegeräten üblicherweise über Druckgasflaschen, Gasgeneratoren oder mittels Adsorbentien. Nachteilig bei diesen Verfahren sind dabei der erforderliche Kosten- und Geräteaufwand sowie bei der Verwendung von Adsorbentien der geringe Reinheitsgrad des Sauerstoffs.

Aus DE 20 2012 102 724 U1 ist ein Analysegerät mit einem thermischen Aufschlusssystem bekannt geworden, das eine Einrichtung zur direkten Erzeugung von Sauerstoff aus Umgebungsluft umfasst. Hierzu dient ein keramisches Material mit Perowskitstruktur, das beispielsweise als Membran oder Granulat ausgebildet sein kann, und das bei hohen Temperaturen eine Sauerstoffionen-Leitfähigkeit aufweist. Diese Sauerstoffionen-Leitfähigkeit erlaubt eine Abtrennung des Sauerstoffs von den übrigen Bestandteilen der Umgebungsluft, indem Sauerstoff selektiv durch das keramische Material transportiert wird. Um das Perowskit-Material auf die für den Sauerstofftransport erforderliche Temperatur zu heizen, soll die Prozesswärme des Analysatorofens ausgenutzt werden, so dass im Wesentlichen keine zusätzliche Energie erforderlich ist. Es ist jedoch keine konkrete Ausgestaltung angegeben, mit der eine ausreichende Temperatur des Perowskit-Materials unter Verzicht auf eine zusätzliche Energiequelle erreichbar ist.

Der vorliegenden Erfindung liegt die Aufgabe zugrunde, ein Analysegerät der eingangs genannten Art anzugeben, welches bei kompakter Bauweise eine energiesparende Sauerstofferzeugung erlaubt.

Diese Aufgabe wird gelöst durch ein Analysegerät mit den in Anspruch 1 genannten Merkmalen. Vorteilhafte Ausgestaltungen der Erfindung sind in den abhängigen Ansprüchen angegeben.

Das erfindungsgemäße Analysegerät zur Bestimmung einer von der Konzentration eines oder mehrerer, insbesondere oxidierbarer, Inhaltsstoffe einer Probe abhängigen Messgröße, umfasst:
- einen Aufschlussreaktor mit einer Sauerstoffzuführung zur Einleitung von Sauerstoff in den Aufschlussreaktor und einem den Aufschlussreaktor mit einer Messeinrichtung verbindenden Gasauslass;
- eine Heizvorrichtung zur Heizung des Aufschlussreaktors auf eine vorgegebene Betriebstemperatur;
- eine Sauerstofferzeugungseinrichtung umfassend mindestens eine sauerstoffpermeable Membran, insbesondere aus einem keramischen Material;
- ein Gehäuse, in welchem ein den Aufschlussreaktor, die Heizvorrichtung und die Sauerstofferzeugungseinrichtung umgebendes Isolierrohr angeordnet ist; und
- ein Speisegasleitungssystem zur Zuleitung eines Speisegases zu der mindestens einen Membran der Sauerstofferzeugungseinrichtung, wobei das Speisegasleitungssystem einen die mindestens eine Membran umgebenden Reaktionsraum umfasst und mit mindestens einem zur Umgebung des Analysegeräts offenen Einströmkanal verbunden ist;
dadurch gekennzeichnet, dass innerhalb des Isolierrohrs mindestens zwei bezüglich der Rohrachse des Isolierrohrs koaxial angeordnete, den Aufschlussreaktor umgebende, insbesondere rohrförmige, Zwischenwände angeordnet sind, welche einen zwischen dem Aufschlussreaktor und dem Isolierrohr angeordneten Zwischenraum in das Speisegasleitungssystem bildende Ringkammern unterteilen, welche durch Überströmöffnungen miteinander verbunden sind.

Durch den Einströmkanal kann Luft aus der Umgebung des Gehäuses des Analysegeräts über das Speisegasleitungssystem dem Reaktionsraum als Speisegas zugeführt werden. Dadurch, dass das Speisegasleitungssystem durch innerhalb des Isolierrohrs angeordnete Ringkammern gebildet und mithin im Wesentlichen vollständig innerhalb des Isolierrohrs angeordnet ist, kann das durch den Einströmkanal in das Speisegasleitungssystem eintretende Speisegas mittels der Heizvorrichtung des Analysegeräts so weit aufgeheizt werden, dass eine zusätzliche Heizung für die Membran nicht benötigt wird. Vielmehr reicht die durch Konvektion mittels des Speisegases zur Membran transportierte Wärme zusammen mit der zur Membran gelangenden Wärmestrahlung der Heizvorrichtung aus, das Keramikmaterial der Membran auf die für eine ausreichende Sauerstoffionenleitung erforderliche Temperatur aufzuheizen. Besonders vorteilhaft ist, dass für die Erwärmung des Speisegases auch keine zusätzlichen Mittel zur Erwärmung des Speisegasstroms, z.B. eine Zusatzheizung oder ein Wärmetauscher, verwendet oder benötigt werden. Indem der Gasstrom allein mittels der für den thermischen Aufschluss verwendeten Heizung des Analysegeräts erwärmt wird, ist ein energiesparender Betrieb des Analysegeräts mit geringem Bauraumbedarf realisiert.

Eine erste der Ringkammern kann den die mindestens eine Membran beinhaltenden Reaktionsraum bilden. Eine zweite der Ringkammern kann als Einströmkammer des Speisegasleitungssystems dienen und mit dem Einströmkanal des Speisegasleitungssystems verbunden sein oder diesen bilden.

Eine der Ringkammern, insbesondere die als Reaktionsraum dienende Ringkammer, kann über mindestens eine durch die Wand des Gehäuses geführte Ausströmöffnung mit der Umgebung des Gehäuses verbunden sein.

Die Ausströmöffnung dient als Auslass für das als Retentat der Sauerstofferzeugungseinrichtung anfallende Speisegas und ist vorzugsweise aus dem Isolierrohr herausgeführt.

Die Ausströmöffnung kann mindestens einen in einer das Isolierrohr umgebenden Gehäusewandung des Gehäuses gebildeten Kanal umfassen, wobei die Gehäusewandung dazu ausgestaltet ist, durch den Kanal strömendes Speisegas abzukühlen. Beispielsweise kann die Gehäusewandung aus einem Material hoher Wärmeleitfähigkeit bestehen und mit zusätzlichen aktiven oder passiven Kühlelementen, wie z.B. Kühlrippen als passiven Kühlelementen, ausgestattet sein, um eine ausreichende Abkühlung des von der Sauerstofferzeugungseinrichtung kommenden, Speisegases bzw. Speisegas-Retentats zu gewährleisten, so dass dieses gefahrlos in die Umgebung abgegeben werden kann.

Vorteilhaft ist eine Ausgestaltung, bei der die Öffnung des Einströmkanals zur Umgebung des Gehäuses in einem unteren Bereich des Gehäuses, beabstandet von einer oder mehreren in den oberen Bereich der Einströmkammer mündenden Überströmöffnungen, die die Einströmkammer mit einer weiteren Ringkammer des Speisegasleitungssystems verbinden, angeordnet sind. Diese Ausgestaltung bewirkt aufgrund eines innerhalb der Einströmkammer auftretenden Kamineffektes eine thermische Konvektion, die durch den Einströmkanal einströmende kühle Luft, welche in der Einströmkammer mittels der Heizvorrichtung des Analysegerätes aufgeheizt wird, über die Überströmöffnung in die weiteren Kammern des Speisegasleitungssystems transportiert.

In einer vorteilhaften Weiterbildung dieser Ausgestaltung mündet die Ausströmöffnung, in einen oberen Bereich der bezogen auf die Strömungsrichtung eines das Speisegasleitungssystem im Betrieb des Analysegeräts durchströmenden Speisegasstromes letzten Ringkammer des Speisegasleitungssystems, bei der es sich beispielsweise um die als Reaktionsraum dienende Ringkammer handeln kann. Diese Ringkammer ist über eine oder mehrere in einem unteren Bereich der Ringkammer angeordneten Überströmöffnungen mit einer weiteren Ringkammer des Speisegasleitungssystems verbunden, über die ihr das Speisegas zugeleitet wird. In dieser Ausgestaltung wird ebenfalls ein innerhalb der äußersten Ringkammer auftretender Kamineffekt zum Transport von Speisegas über die Ausströmöffnung in die Umgebung genutzt.

Die rohrförmigen Zwischenwände können vorteilhaft aus einem Material gebildet sein, das für Wärmestrahlung, insbesondere im Infrarotbereich, mindestens teilweise transparent ist, so dass die von der Heizvorrichtung ausgehende Wärmestrahlung zur Aufheizung der Membran beitragen kann. Als Material kommt beispielsweise Quarzglas in Frage.

Das Speisegasleitungssystem kann einen die Heizvorrichtung umgebenden Heizungsraum aufweisen. Beispielsweise kann eine der Ringkammern des Speisegasleitungssystems als Heizungsraum dienen, so dass ein durch das Speisegasleitungssystem strömender Speisegasstrom die Heizvorrichtung umströmt. Alternativ kann die Heizvorrichtung aber auch außerhalb des Speisegasleitungssystems angeordnet sein. Die Heizvorrichtung kann beispielsweise einen spiralförmig um den Aufschlussreaktor verlaufenden, insbesondere innerhalb des Heizungsraums angeordneten, elektrischen Heizleiter umfassen.

Es sind unterschiedliche Anordnungen der Ringkammern denkbar. Weist das Speisegasleitungssystem einen Heizungsraum auf, kann die den Reaktionsraum bildende Ringkammer beispielsweise die den Heizungsraum bildende Ringkammer umschließen. Alternativ ist es auch möglich, dass die den Heizungsraum bildende Ringkammer die den Reaktionsraum bildende Ringkammer umschließt. Zusätzlich kann das Speisegasleitungssystem weitere koaxial angeordnete Ringkammern umfassen, die jeweils gegebenenfalls durch weitere, koaxial angeordnete rohrförmige Zwischenwände von den weiteren Ringkammern abgeteilt und durch Überströmöffnungen miteinander verbunden sind.

Das Speisegas ist vorzugsweise Luft aus der Umgebung des Analysegeräts.

Die erwähnte Messeinrichtung kann Bestandteil des Analysegeräts sein. Sie umfasst mindestens einen spezifischen Detektor zur Erfassung des Gehalts einer oder mehrerer vorgegebener Verbindungen, z.B. CO₂ oder NOₓ, in einem den Aufschlussreaktor über den Gasauslass verlassenden Gasstrom. Zusätzlich kann die Messeinrichtung eine Auswerteeinheit umfassen oder mit einer externen Auswerteeinheit verbindbar ausgestaltet sein. Beispielsweise kann das Analysegerät als spezifischen Detektor zur Bestimmung eines TOC-Wertes einen Infrarotdetektor umfassen, der dazu ausgestaltet ist, ein von dem CO₂-Gehalt des Gasstroms abhängiges Messsignal zu erzeugen. Alternativ oder zusätzlich kann das Analysegerät als spezifischen Detektor zur Bestimmung eines TN_{b}-Wertes einen Chemilumineszenz-Detektor (CLD-Detektor) umfassen. Der spezifische Detektor zur Bestimmung des TN_{b}-Wertes kann auch dazu ausgestaltet sein, ein auf Infrarot-Detektion oder einer elektrochemischen Messung basierendes Messsignal zu erzeugen. Handelt es sich bei dem Analysegerät um einen Elementaranalysator kann die Messeinrichtung einen für das zu bestimmende Element, z.B. Kohlenstoff, Schwefel, Stickstoff, Wasserstoff und/oder Chlor, spezifischen Detektor umfassen. Solche Detektoren sind im Stand der Technik an sich bekannt.

Die Sauerstofferzeugungseinrichtung kann mit der Sauerstoffzuführung des Aufschlussreaktors zur Einleitung von durch die Sauerstofferzeugungseinrichtung erzeugten Sauerstoffes in den Aufschlussreaktor verbunden sein, um im Betrieb des Analysegeräts den erzeugten Sauerstoff direkt in den Aufschlussreaktor einzuleiten.

Die Membran der Sauerstofferzeugungseinrichtung kann als einseitig geschlossene Membranöhre ausgestaltet sein, welche eine dem Reaktionsraum zugewandte äußere Retentatseite und eine dem Inneren der Röhre zugewandte innere Permeatseite aufweist. Herrscht an der Permeatseite ein geringerer Sauerstoffpartialdruck als an der Retentatseite, wird Sauerstoff aus dem im Reaktionsraum vorliegenden Speisegas durch die Membran ins Innere der Membranröhre transportiert. Je nach der für den Aufschluss benötigten Sauerstoffmenge, kann die Sauerstofferzeugungseinrichtung mehrere als Membranröhren ausgestaltete Membranen aufweisen. In einer Ausgestaltung kann das Analysegerät mehrere Membranen, insbesondere mehrere derart als Membranröhren ausgestaltete Membranen, aufweisen.

Das Innere der Membranröhre kann über eine Pumpe mit der Sauerstoffzuführung des Aufschlussreaktors verbunden sein. Die Pumpe kann dazu ausgestaltet sein, im Inneren der Röhre einen Unterdruck bezüglich des in dem Reaktionsraum herrschenden Druckes zu erzeugen, so dass an der Permeatseite ein geringerer Sauerstoffpartialdruck herrscht als an der Retentatseite der Membran.

Die Durchmesser und Längen der Ringkammern sowie der Durchfluss des Speisegases durch das Speisegasleitungssystem sind auf die Länge der Membranröhre so abgestimmt, dass im Betrieb des Analysegeräts die Röhre über die gesamte Länge ihres in den Reaktionsraum ragenden Abschnitts eine Temperatur aufweist, welche größer ist als ein vorgegebener Temperaturschwellenwert, der einer Mindestbetriebstemperatur der Membran entspricht. Sind mehrere Membranen, insbesondere als Membranröhren gebildete Membranen vorhanden, gilt dasselbe für alle Membranen. Die Mindestbetriebstemperatur entspricht einer Temperatur, bei der eine ausreichende Sauerstoffionen-Leitfähigkeit des keramischen Membranmaterials auftritt. Bei einem keramischen, sauerstoffleitenden Perowskitmaterial liegt diese Temperatur vorzugsweise über 500°C, besonders bevorzugt über 800 °C. In einer Ausgestaltung beträgt die Ringbreite des Querschnitts des Reaktionsraums, d.h. die Differenz zwischen dem inneren Radius und dem äußeren Radius des Querschnitts, das 0,01- bis 0,1-fache der Länge des sich innerhalb des Reaktionsraums befindlichen Abschnitts der die Membran bildenden Röhre. Typischerweise erstreckt sich die Membranröhre nahezu über die gesamte Länge des Reaktionsraums, so dass die Ringbreite des Querschnitts des Reaktionsraums ebenfalls etwa das 0,01- bis 0,1-fache der Länge des Reaktionsraums beträgt.

Die Erfindung wird im Folgenden anhand des in der Figur dargestellten Ausführungsbeispiels näher erläutert. Es zeigen:
- Fig. 1: eine schematische Darstellung eines Analysegeräts;
- Fig. 2: eine schematische Längsschnitt-Darstellung des Aufschlussreaktors, der Sauerstofferzeugungseinrichtung und dem Speisegasleitungssystem des in Fig. 1 dargestellten Analysegeräts.

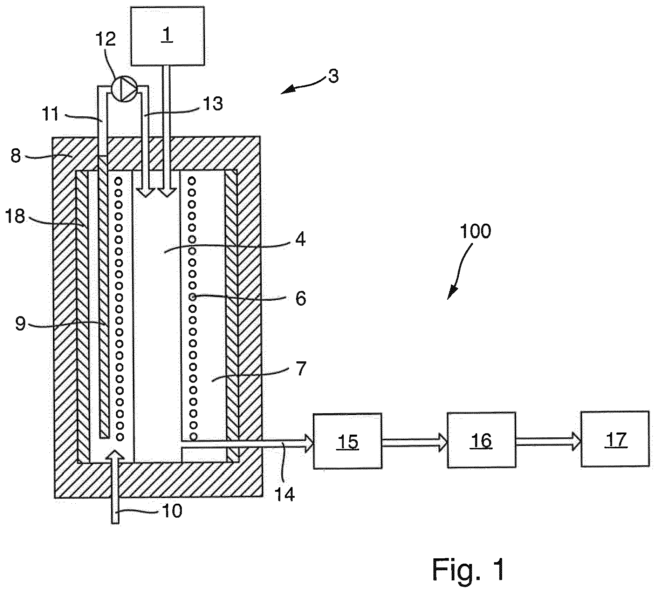

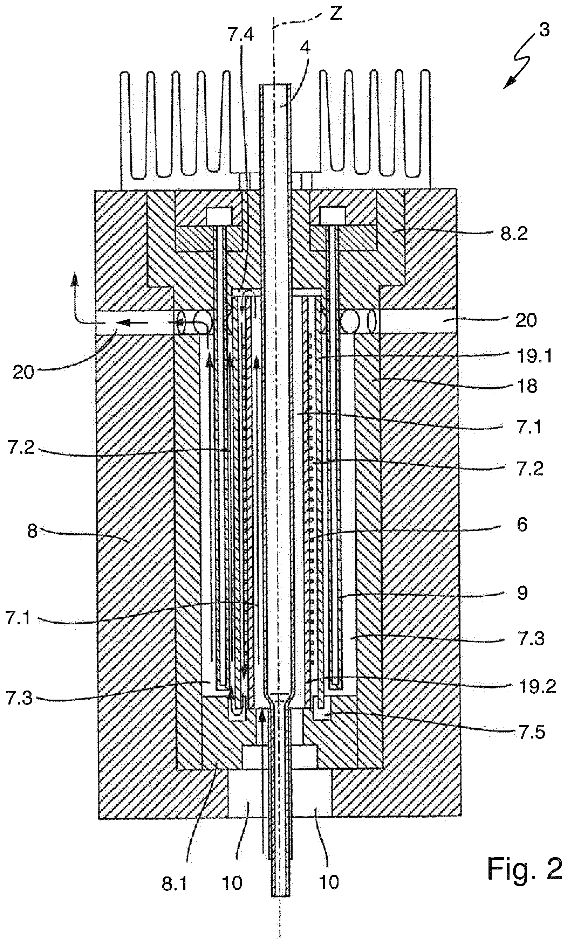

Das Analysegerät 100 weist ein Probendosiersystem 1 auf, über das eine zu untersuchende Substanz einem thermischen Aufschlusssystem 3 zuführbar ist. Das Aufschlusssystem 3 umfasst einen rohrförmigen Aufschlussreaktor 4, welcher mittels einer Heizvorrichtung 6 beheizbar ist. Die Heizvorrichtung 6 ist im vorliegenden Beispiel als elektrische Widerstandsheizung ausgestaltet, welche einen spiralförmig um den Aufschlussreaktor verlaufenden Heizleiter umfasst. Das Aufschlusssystem 3 ist in einem Gehäuse 8 untergebracht, in dem auch eine Sauerstofferzeugungseinrichtung mit mehreren sauerstoffpermeablen Membranen 9 (der Übersichtlichkeit halber ist in Fig. 1 nur eine Membran dargestellt) und einem in Fig. 1 zunächst nicht näher dargestellten, den Aufschlussreaktor 4 umgebenden Speisegasleitungssystem 7 (vgl. Fig. 2) angeordnet ist. Der Aufschlussreaktor 4, die Heizvorrichtung 6 und das Speisegasleitungssystem 7 sind mittels eines diese umgebenden Isolierrohrs 18 gegenüber dem Gehäuse 8 thermisch isoliert.

Die Membranen 9 sind im vorliegenden Beispiel als aus einem keramischen Material mit Sauerstoffionen-Leitfähigkeit gebildete Röhren ausgestaltet. Geeignete keramische Materialien sind beispielsweise Oxide mit Perowskitstruktur. Perowskite sind ternäre Oxide mit der Gitterstruktur des ABO₃-Typs. Oberhalb einer materialabhängigen Mindestbetriebstemperatur, die zwischen 500 und 1000 °C liegen kann, weisen diese Materialien eine simultane elektrische Leitfähigkeit und Sauerstoffionen-Leitfähigkeit auf. Somit ist es möglich, Sauerstoff durch eine gasdichte keramische Membran zu transportieren, wobei eine Abtrennung des Sauerstoffs von anderen Bestandteilen eines der Membran zugeführten Speisegases, das zum Beispiel Luft sein kann, erfolgt. Herrscht auf der Außenseite der Röhren (auch als Retentatseite bezeichnet) ein höherer Sauerstoffpartialdruck als auf der dem Inneren der Röhren zugewandten Innenseite (auch als Permeatseite bezeichnet), so werden bei Temperaturen oberhalb der Mindestbetriebstemperatur der Membran, Sauerstoffmoleküle an der Retentatseite zu negativ geladenen Sauerstoffionen reduziert, Sauerstoffionen von der Retentatseite zur Permeatseite durch die Membran transportiert und an der Permeatseite Sauerstoffionen zu molekularem Sauerstoff oxidiert. Auf diese Weise wird Sauerstoff durch die Membran transportiert und von den auf der Retentatseite verbleibenden übrigen Bestandteilen des Speisegases getrennt. Geeignete Materialien sind beispielsweise perowskitartige Oxide wie Ba₁₋ₓSrₓCo_{1-y}Fe_{y}O_{3-δ}, speziell z.B. Ba_{0,5}Sr_{0,5}Co_{0,8}Fe_{0,2}, oder La₁₋ₓSrₓCo_{1-y}Fe_{y},O_{3-δ}, speziell z.B. La_{0,2}Sr_{0,8}Co_{0,5}Fe_{0,5}O_{3-δ}, sowie Nickel/Kobalt-Perowskit-Oxid mit der Zusammensetzung La_{0,5}Sr_{0,5}Co_{0,8}Ni_{0,2}O_{3-δ}.

Die als Röhren ausgestalteten Membranen 9 sind an einem Ende verschlossen. Ihr entgegengesetztes Ende ist über eine Gasleitung 11 mit einer Pumpe 12 verbunden, welche dazu ausgestaltet ist, im Inneren der Röhren einen Unterdruck zu erzeugen, so dass zwischen Retentatseite und Permeatseite das für den Sauerstofftransport erforderliche Sauerstoffpartialdruck-Gefälle entsteht. Als Speisegas wird im hier gezeigten Beispiel Luft aus der Umgebung des Analysegeräts 100 über den Einströmkanal 10 in das Speisegasleitungssystem 7 eingeleitet. Es können auch mehrere Einströmkanäle vorhanden sein, hier ist der Übersichtlichkeit halber nur ein Einströmkanal dargestellt.

Mittels der Pumpe 12 wird der auf der Permeatseite der Membranen 9 anfallende gasförmige Sauerstoff über die Gaszuleitung 13 in den Aufschlussreaktor 4 eingeleitet. Dabei dient der Sauerstoff gleichzeitig als Trägergas und als Oxidationsmittel für die Inhaltsstoffe der in den Aufschlussreaktor über das Probendosiersystem 1 eingebrachten Probe. Der Aufschluss wird bei einer Temperatur zwischen 500 und 1000 °C durchgeführt, die mittels der Heizungsvorrichtung 6 erreicht wird. Der Aufschlussreaktor weist neben der Gaszuleitung 13 auch einen Gasauslass 14 auf, der den Aufschlussreaktor 4 mit einem Trockner 15 verbindet. Der Trockner 15 ist über eine Gasleitung mit einem Detektor 16 verbunden, der dazu ausgestaltet ist, ein von der zu bestimmenden Messgröße abhängiges Messsignal auszugeben. Im vorliegenden Beispiel eines TOC-Analysegeräts kann es sich bei dem Detektor 16 um einen Infrarotdetektor handeln, der dazu ausgestaltet ist, ein vom CO₂-Gehalt des aus dem Gasauslass 14 über den Trockner 15 dem Detektor 16 zugeleiteten Gasstrom abhängiges Messsignal zu erzeugen. Der Detektor 16 ist mit einer Auswerteeinheit 17 verbunden, die dazu ausgestaltet ist, das Messsignal des Detektors 16 zu erfassen und anhand des Messsignals einen Messwert der Messgröße, hier des TOC-Werts der Probe, zu ermitteln. Bei der Auswerteeinheit 17 kann es sich beispielsweise um eine elektronische Datenverarbeitungseinrichtung, insbesondere einen PC, handeln, der ein der TOC-Bestimmung dienendes Auswerteprogramm umfasst und ausführen kann.

Die Sauerstofferzeugung kann nach Bedarf erfolgen, wobei die Sauerstoffflüsse an die jeweiligen Erfordernisse angepasst werden können. Die Steuerung der Sauerstofferzeugung kann durch Auslegung des Membranreaktors, insbesondere durch die Anzahl der verwendeten Membranen, und durch Steuerung der Betriebsparameter, insbesondere der Partialdruckdifferenz zwischen Permeatseite und Retentatseite der Membranen 9, vorgenommen werden. Das Analysegerät 100 benötigt und verwendet keine zusätzlichen Heizmittel oder Wärmetauscher zur Heizung der Membranen 9 auf eine Temperatur oberhalb der Mindestbetriebstemperatur. Die zur Heizung des Aufschlussreaktors 4 dienende Heizvorrichtung 6 dient gleichzeitig zur Heizung der Membranen 9.

In Fig. 2 ist eine mögliche Ausgestaltung des in dem thermischen Aufschlusssystem 3 des in Fig. 1 dargestellten Analysegeräts 100 integrierten Speisegasleitungssystems 7 schematisch im Detail dargestellt. Mit dem in Fig. 1 dargestellten System identische Komponenten sind mit gleichen Bezugszeichen versehen. Das Aufschlusssystem 3 ist im wesentlichen zylindersymmetrisch aufgebaut und besitzt eine Zylindersymmetrieachse Z, die mit der Rohrachse des Aufschlussreaktors 4 und des Isolierrohrs 18 zusammenfällt. Das Aufschlusssystem 3 weist ein zylindrisches Gehäuse 8 auf, das den Aufschlussreaktor 4 und das Speisegasleitungssystem 7 umgibt. Das zylindrische Gehäuse 8 ist an seinen einander gegenüberliegenden Stirnseiten durch Einsätze 8.1, 8.2 verschlossen. Der Aufschlussreaktor 4 und das Speisegasleitungssystem 7 sind gegenüber dem Gehäuse 8 mittels eines Isolierrohrs 18 thermisch isoliert. Zwei den Aufschlussreaktor 4 umgebende rohrförmige, koaxial bezüglich der Zylinderachse Z angeordnete Zwischenwände 19.1, 19.2 unterteilen den den Aufschlussreaktor 4 umgebenden, von dem Isolierrohr 18 und dem Gehäuse 8 umgebenen ringförmigen Raum in drei Ringkammern 7.1, 7.2, 7.3. Die rohrförmigen Zwischenwände 19.1, 19.2 greifen an ihren Enden derart in die Einsätze 8.1, 8.2 ein, dass ein Gastransport von einer Ringkammer zur anderen nur über die Überströmkanäle 7.4, 7.5 möglich ist. Die Zwischenwände 19.1, 19.2 bestehen aus einem Material, das Wärmestrahlung der Heizvorrichtung 6 mindestens teilweise transmittiert. Als Material für die Zwischenwände 19.1, 19.2 kommt beispielsweise Quarzglas in Frage.

Im Einsatz 8.2 sind die als einseitig verschlossene Röhren ausgestalteten Membranen 9 befestigt. Sie erstrecken sich parallel zur Zylinderachse Z durch die äußere der drei Ringkammern 7.3, wobei die Länge des innerhalb der Ringkammer 7.3 angeordneten Abschnitts der Membranen 9 im Wesentlichen der axialen Längserstreckung der Ringkammer 7.3 entspricht. Diese durch das Isolierrohr 18 und die äußere Zwischenwand 19.1 begrenzte Ringkammer 7.3 bildet den Reaktionsraum des Speisegasleitungssystems 7, in dem Sauerstoff auf der (äußeren) Retentatseite der Membranen 9 zu Sauerstoffionen reduziert wird, welche durch die Membranen 9 auf die (innere) Permeatseite der Membranen 9 transportiert werden können, wie weiter oben beschrieben. Die äußere Ringkammer 7.3 ist über radial bezüglich der Zylinderachse Z durch die Wand des Gehäuses 8 verlaufende Ausströmöffnungen 20 mit der Umgebung verbunden. Diese Ausströmöffnungen 20 sind als Kanäle ausgestaltet und dienen als Retentatauslass.

In der zwischen der äußeren Zwischenwand 19.1 und der inneren Zwischenwand 19.2 angeordnete Ringkammer 7.2 ist die Heizvorrichtung 6 angeordnet, im hier gezeigten Beispiel ist in dieser Ringkammer 7.2 ein spiralförmig die Zwischenwand 19.2 und gleichzeitig auch den Aufschlussreaktor 4 umgebender Heizleiter angeordnet. Die Ringkammer 7.2 bildet somit den Heizungsraum des Speisegasleitungssystems 7. Die innerste, von der Zwischenwand 19.2 und der Wand des Aufschlussreaktors 4 begrenzte Ringkammer 7.1 ist über die Einströmkanäle 10 des Speisegasleitsystems 7 mit der Umgebungsatmosphäre verbunden.

Im Betrieb des Analysegeräts 100 bildet sich ein Speisegasstrom zwischen den Einströmöffnungen 10 und den als Retentatauslass dienenden Ausströmöffnungen 20 aus. Dieser Speisegasstrom verläuft in Pfeilrichtung zunächst durch die innerste Ringkammer 7.1 zwischen dem Aufschlussreaktor 4 und der Zwischenwand 19.2 der Heizungskammer, wo die einströmende kalte Luft bereits erwärmt wird. Über die Überströmkanäle 7.4 tritt der Speisegasstrom in den Heizungsraum 7.2 ein und wird dort über die Windungen des Heizleiters geführt, so dass das Speisegas weiter erwärmt wird. Der Gasstrom verläuft weiter über die Überströmkanäle 7.5 in den Reaktionsraum 7.3, wo das erwärmte Speisegas durch Konvektion zusammenwirkend mit der durch die Zwischenwände 19.1, 19.2 von der Heizvorrichtung 9 zu den Membranen 9 gelangende Wärmestrahlung die Membranen 9 über ihre gesamte im Reaktionsraum 7.3 angeordnete Länge auf eine Temperatur erwärmt, die über ihrer Mindestbetriebstemperatur liegt. Im Inneren der röhrenförmigen Membranen 9 wird mittels der Pumpe 12 ein Unterdruck erzeugt, so dass, wie bereits beschrieben, ein Sauerstofftransport durch die Membran 9 erfolgt. Das im Reaktionsraum zurückbleibende Gasgemisch, das Retentat, wird durch die Ausströmöffnungen 20 in die Umgebung entlassen. Beim Durchströmen der Ausströmöffnungen 20 kühlt das Gasgemisch wieder ab, so dass es problemlos in die Umgebungsatmosphäre abgegeben werden kann.

Die Länge und Ringbreite der Ringkammern des Speisegasleitungssystems, die Länge und Anzahl der Membranen und der Fluss des Speisegasstroms sind vorzugsweise derart aufeinander abgestimmt, dass die Membranen über die gesamte Länge ihrer durch den Reaktionsraum verlaufenden Abschnitte ihre Mindestbetriebstemperatur erreichen. Im vorliegenden Beispiel hat der kreisringförmige Querschnitt der äußersten Ringkammer 7.3, also des die Membranen 9 enthaltenden Reaktionsraums, beispielsweise eine Ringbreite (=Differenz zwischen Außen- und Innendurchmesser) von 5 bis 20 mm bei einer Längserstreckung des Raums zwischen 100 bis 500 mm. Der Querschnitt des durch die mittlere Ringkammer 7.2 gebildeten Heizungsraums hat im vorliegenden Beispiel eine Ringbreite von 1 bis 5 mm bei gleicher Längserstreckung wie die äußerste Ringkammer 7.3. Die innerste Ringkammer 7.1 kann ähnliche Abmessungen aufweisen wie die mittlere Ringkammer 7.2, d.h. einen Querschnitt mit einer Ringbreite von 1 bis 5 mm und eine Länge zwischen 100 und 500 mm. Mit dem beschriebenen Aufbau kann mit einer Anzahl von 6 bis 12 Membranen 9 ein Sauerstoff-Fluss von ca. 50 ml/ min bis 10 l/min erzielt werden.

Von dem hier dargestellten Ausführungsbeispiel sind Abwandlungen denkbar, die ebenfalls Gegenstand der hier beschriebenen Erfindung sind. Beispielsweise ist es möglich, die Heizvorrichtung in einer weiter außen liegenden Ringkammer anzuordnen und die Membranen in einer von dem ringförmigen Heizungsraum umgebenen weiter innen liegenden Ringkammer anzuordnen. Der Gasstrom verläuft auch in dieser Ausgestaltung von einer oder mehreren Einströmkanälen durch den Heizungsraum in den Reaktionsraum. Diese Anordnung ist vorteilhaft, wenn der thermische Aufschluss in Gegenwart eines Katalysators durchzuführen ist, was dazu führen kann, dass der Aufschlussreaktor auf eine Temperatur zu heizen ist, die sogar unterhalb der Mindestbetriebstemperatur der Membranen liegt. Es ist in weiteren Ausgestaltungsvarianten außerdem möglich, weitere Ringkammern oder einen anderen Verlauf des Speisegasstromes vorzusehen.

## Patentansprüche

1. Analysegerät (100) zur Bestimmung einer von der Konzentration eines oder mehrerer, insbesondere oxidierbarer, Inhaltsstoffe einer Probe abhängigen Messgröße, umfassend:
- einen Aufschlussreaktor (4) mit einer Sauerstoffzuführung zur Einleitung von Sauerstoff in den Aufschlussreaktor (4) und einem den Aufschlussreaktor (4) mit einer Messeinrichtung verbindenden Gasauslass (14);
- eine Heizvorrichtung (6) zur Heizung des Aufschlussreaktors (4) auf eine vorgegebene Betriebstemperatur;
- eine Sauerstofferzeugungseinrichtung umfassend mindestens eine sauerstoffpermeable Membran (9), insbesondere aus einem keramischen Material;
- ein Gehäuse (8), in welchem ein den Aufschlussreaktor (4), die Heizvorrichtung (6) und die Sauerstofferzeugungseinrichtung umgebendes Isolierrohr (18) angeordnet ist; und
- ein Speisegasleitungssystem (7) zur Zuleitung eines Speisegases zu der mindestens einen Membran (9) der Sauerstofferzeugungseinrichtung, wobei das Speisegasleitungssystem (7) einen die mindestens eine Membran (9) umgebenden Reaktionsraum umfasst und mit mindestens einem zur Umgebung des Analysegeräts (100) offenen Einströmkanal (10) verbunden ist;
**dadurch gekennzeichnet, dass** innerhalb des Isolierrohrs (18) mindestens zwei bezüglich der Rohrachse des Isolierrohrs (18) koaxial angeordnete, den Aufschlussreaktor (4) umgebende, insbesondere rohrförmige, Zwischenwände (19.1, 19.2) angeordnet sind, welche einen zwischen dem Aufschlussreaktor (4) und dem Isolierrohr (18) angeordneten Zwischenraum in das Speisegasleitungssystem bildende Ringkammern (7.1, 7.2, 7.3) unterteilen, welche durch Überströmöffnungen miteinander verbunden sind.

2. Analysegerät (100) nach Anspruch 1,
wobei eine der Ringkammern (7.3) den Reaktionsraum bildet und über mindestens eine durch die Wand des Gehäuses (8) geführte Ausströmöffnung (20) mit der Umgebung des Gehäuses (8) verbunden ist.

3. Analysegerät (100) nach Anspruch 2,
wobei die Ausströmöffnung (20) mindestens einen in einer das Isolierrohr (18) umgebenden Gehäusewandung gebildeten Kanal umfasst, wobei die Gehäusewandung dazu ausgestaltet ist, durch den Kanal aus dem Speisegasleitungssystem (7) ausströmendes Speisegas abzukühlen.

4. Analysegerät (100) nach einem der Ansprüche 1 bis 3,
wobei eine der Ringkammern (7.1) als Einströmkammer ausgestaltet ist, in die der Einströmkanal (10) mündet, und wobei eine Öffnung des Einströmkanals (10) zur Umgebung des Gehäuses (8) in einem unteren Bereich des Gehäuses (8) und beabstandet von einer oder mehreren in einen oberen Bereich der Einströmkammer mündenden Überströmöffnungen, die die Einströmkammer mit einer weiteren Ringkammer (7.2) des Speisegasleitungssystems (7) verbinden, angeordnet sind.

5. Analysegerät (100) nach einem der Ansprüche 2 bis 4,
wobei die Ausströmöffnung (20) in einen oberen Bereich der bezogen auf eine Strömungsrichtung eines das Speisegasleitungssystem (7) im Betrieb des Analysegeräts durchströmenden Speisegasstromes letzten Ringkammer (7.3) des Speisegasleitungssystems (7) mündet, und wobei diese Ringkammer (7.3) über in einem unteren Bereich der Ringkammer (7.3), beabstandet von der Ausströmöffnung (20) angeordnete Überströmöffnungen mit einer weiteren Ringkammer (7.2) des Speisegasleitungssystems (7) verbunden ist.

6. Analysegerät (100) nach einem der Ansprüche 1 bis 5,
wobei die rohrförmigen Zwischenwände (19.1, 19.2) aus einem Material gebildet sind, das für Wärmestrahlung, insbesondere Infrarotstrahlung, mindestens teilweise transparent ist.

7. Analysegerät (100) nach einem der Ansprüche 1 bis 6,
wobei die Heizvorrichtung (6) in einer der Ringkammern (7.2) des Speisegasleitungssystems (7) angeordnet ist.

8. Analysegerät (100) nach einem der Ansprüche 1 bis 7,
wobei die Sauerstofferzeugungseinrichtung mit der Sauerstoffzuführung des Aufschlussreaktors (4) zur Einleitung von durch die Sauerstofferzeugungseinrichtung erzeugten Sauerstoffes in den Aufschlussreaktor (4) verbunden ist.

9. Analysegerät (100) nach einem der Ansprüche 1 bis 8,
wobei die Membran (9) als einseitig geschlossene Membranröhre ausgestaltet ist, welche eine dem Reaktionsraum zugewandte äußere Retentatseite und eine dem Inneren der Membranröhre zugewandte innere Permeatseite aufweist.

10. Analysegerät (100) nach Anspruch 9,
wobei das Innere der Membranröhre über eine Pumpe (12) mit der Sauerstoffzuführung des Aufschlussreaktors verbunden ist, und wobei die Pumpe (12) dazu ausgestaltet ist, innerhalb der Membranröhre einen Unterdruck bezüglich des in dem Reaktionsraum herrschenden Druckes zu erzeugen.

11. Analysegerät (100) nach Anspruch 9 oder 10,
wobei Durchmesser und Längen der Ringkammern (7.1, 7.2, 7.3) sowie der Durchfluss des Speisegases durch das Speisegasleitungssystem (7) so auf die Länge des innerhalb des Reaktionsraums angeordneten Abschnitts der Membranröhre abgestimmt sind, dass im Betrieb des Analysegeräts die Membranröhre über die gesamte Länge ihres innerhalb des Reaktionsraums angeordneten Abschnitts eine Temperatur aufweist, welche größer ist als ein vorgegebener Temperaturschwellenwert, der einer Mindestbetriebstemperatur der Membran entspricht.

12. Analysegerät (100) nach einem der Ansprüche 9 bis 11,
wobei die Ringbreite des Querschnitts des Reaktionsraums zwischen dem 0,01- und dem 0,1-fachen der Länge des sich innerhalb des Reaktionsraums befindlichen Abschnitts der Membranröhren beträgt.

## Claims

1. Analyzer (100) for determining a measured variable which depends on the concentration of one or more components, particularly oxidizable components, of a sample, comprising:
- a digestion reactor (4) with an oxygen supply line for the introduction of oxygen into the digestion reactor (4) and a gas outlet (14) that connects the digestion reactor (4) to a measuring apparatus;
- a heating unit (6) designed to heat the digestion reactor (4) to a predefined operating temperature;
- an oxygen generation unit comprising at least one membrane (9) that is permeable to oxygen, particularly a membrane made from a ceramic material;
- a housing (8) in which an insulating tube (18) that surrounds the digestion reactor (4), the heating unit (6) and the oxygen generation unit is arranged; and
- a feed gas conduit system (7) to supply a feed gas to the at least one membrane (9) of the oxygen generation unit, wherein the feed gas conduit system (7) comprises a reaction chamber surrounding the at least one membrane (9) and is connected to at least one admission channel (10) that is open to the environment of the analyzer (100);
**characterized in that**
at least two, particularly tubular, partition walls (19.1, 19.2) that surround the digestion reactor (4) are arranged within the insulating tube (18) in a way that they are coaxially positioned in relation to the tube axis of the insulating tube (18), said partition walls dividing an intermediate space between the digestion reactor (4) and the insulating tube (18) into annular chambers (7.1, 7.2, 7.3) forming the feed gas conduit system, said chambers being interconnected by overflow openings.

2. Analyzer (100) as claimed in Claim 1,
wherein one of the annular chambers (7.3) forms the reaction chamber and is connected to the environment of the housing (8) via at least one outlet opening (20) through the wall of the housing (8).

3. Analyzer (100) as claimed in Claim 2,
wherein the outlet opening (20) comprises at least one channel formed in a housing wall that surrounds the insulating tube (18), wherein the housing wall is designed to cool feed gas flowing out of the feed gas conduit system (7) via the channel.

4. Analyzer (100) as claimed in one of the Claims 1 to 3,
wherein one of the annular chambers (7.1) is designed as an admission chamber into which the admission channel enters (10), and wherein an opening of the admission channel (10) towards the environment of the housing (8) is arranged in a lower part of the housing (8) at a distance from one or more overflow openings that enter into an upper area of the admission chamber, said overflow openings connecting the admission chamber with another annular chamber (7.2) of the feed gas conduit system (7).

5. Analyzer (100) as claimed in one of the Claims 2 to 4,
wherein the outlet opening (20) enters into an upper area of the last annular chamber (7.3) of the feed gas conduit system (7) considered from the perspective of a flow direction of a feed gas flow flowing through the feed gas conduit system (7) when the analyzer is in operation, and wherein said annular chamber (7.3) is connected to another annular chamber (7.2) of the feed gas conduit system (7) via overflow openings arranged in a lower section of the annual chamber (7.3), situated at a distance from the outlet opening (20).

6. Analyzer (100) as claimed in one of the Claims 1 to 5,
wherein the tubular partition walls (19.1, 19.2) are made from a material that is at least partially transparent for thermal radiation, particularly infrared radiation.

7. Analyzer (100) as claimed in one of the Claims 1 to 6,
wherein the heating unit (6) is arranged in one of the annular chambers (7.2) of the feed gas conduit system (7).

8. Analyzer (100) as claimed in one of the Claims 1 to 7,
wherein the oxygen generation unit is connected to the oxygen supply system of the digestion reactor (4) for the purpose of introducing oxygen generated by the oxygen generation unit into the digestion reactor (4).

9. Analyzer (100) as claimed in one of the Claims 1 to 8,
wherein the membrane (9) is designed as a membrane tube closed on one side, which has an outer retentate side facing towards the reaction chamber and an inner permeate side facing towards the interior of the membrane tube.

10. Analyzer (100) as claimed in Claim 9,
wherein the interior of the membrane tube is connected to the oxygen supply system of the digestion reactor via a pump (12), and wherein the pump is designed to generate, within the membrane tube, a negative pressure in relation to the pressure present in the reaction chamber.

11. Analyzer (100) as claimed in Claim 9 or 10,
wherein the diameters and lengths of the annular chambers (7.1, 7.2, 7.3) as well as the flow of the feed gas through the feed gas conduit system (7) are matched to the length of the section of membrane tube arranged inside the reaction chamber in such a way that, when the analyzer is in operation, the membrane tube presents a temperature over the entire length of the section arranged within the reaction chamber that is greater than a predefined temperature threshold value which corresponds to a minimum operating temperature of the membrane.

12. Analyzer (100) as claimed in one of the Claims 9 to 11,
wherein the annular width of the cross-section of the reaction chamber is between 0.01 and 0.1 times the length of the section of the membrane tube located inside the reaction chamber.

## Revendications

1. Analyseur (100) destiné à la détermination d'une grandeur de mesure dépendant de la concentration d'un ou de plusieurs composants, notamment oxydables, d'un échantillon, comprenant :
- un réacteur digesteur (4) avec une conduite d'alimentation en oxygène pour l'introduction d'oxygène dans le réacteur digesteur (4) et une sortie gaz (14) reliant le réacteur digesteur (4) avec un dispositif de mesure ;
- un dispositif de chauffage (6) destiné au chauffage du réacteur digesteur (4) à une température de service prédéfinie ;
- un dispositif de génération d'oxygène comprenant au minimum une membrane (9) perméable à l'oxygène, notamment constituée d'un matériau en céramique ;
- un boîtier (8), dans lequel sont disposés un tube isolant (18) entourant le réacteur digesteur (4), le dispositif de chauffage (6) et le dispositif de génération d'oxygène ; et
- un système de conduite de gaz d'alimentation (7) pour l'acheminement d'un gaz d'alimentation en direction de l'au moins une membrane (9) du dispositif de génération d'oxygène, le système de conduite de gaz d'alimentation (7) comprenant un espace de réaction entourant l'au moins une membrane (9) et étant reliée avec au moins un canal d'admission (10) ouvert vers l'environnement de l'analyseur (100) ;
**caractérisé**
**en ce que** sont disposées, à l'intérieur du tube isolant (18), au moins deux cloisons (19.1, 19.2), notamment tubulaires, entourant le réacteur digesteur et disposées coaxialement par rapport à l'axe du tube isolant (18), lesquelles cloisons divisent l'espace intermédiaire disposé entre le réacteur digesteur (4) et le tube isolant (18) en chambres annulaires (7.1, 7.2, 7.3) formant le système de conduite de gaz d'alimentation, lesquelles chambres sont reliées entre elles à travers les ouvertures de trop-plein.

2. Analyseur (100) selon la revendication 1,
pour lequel l'une des chambres annulaires (7.3) forme l'espace de réaction et est reliée avec l'environnement du boîtier (8) via au moins une ouverture de sortie (20) acheminée à travers la paroi du boîtier (8).

3. Analyseur (100) selon la revendication 2,
pour lequel l'ouverture de sortie (20) comprend au moins un canal formé par une paroi de boîtier entourant le tube isolant (18), la paroi de boîtier étant conçue de telle sorte à refroidir le gaz d'alimentation sortant du système de conduite de gaz d'alimentation (7) à travers le canal.

4. Analyseur (100) selon l'une des revendications 1 à 3,
pour lequel l'une des chambres annulaires (7.1) est conçue en tant que chambre d'admission (10), dans laquelle débouche le canal d'admission (10), et pour lequel sont disposées une ouverture du canal d'admission (10) dirigée vers l'environnement du boîtier (8), dans une zone inférieure du boîtier (8) et, situées à distance, des ouvertures de trop-plein débouchant dans une zone supérieure de la chambre d'admission, ouvertures qui relient la chambre d'admission avec une autre chambre annulaire (7.2) du système de conduite de gaz d'alimentation (7).

5. Analyseur (100) selon l'une des revendications 2 à 4,
pour lequel l'ouverture de sortie (20) débouche dans une zone supérieure, par rapport à un sens d'écoulement d'un flux de gaz d'alimentation traversant le système de conduite de gaz d'alimentation (7) pendant le fonctionnement de l'analyseur, de la dernière chambre annulaire (7.3) du système de conduite de gaz d'alimentation (7), et pour lequel cette chambre annulaire (7.3) est reliée avec une autre chambre annulaire (7.2) du système de conduite de gaz d'alimentation (7) par l'intermédiaire des ouvertures de trop-plein disposées dans une zone inférieure de la chambre annulaire (7.3), située à distance de l'ouverture de sortie (20).

6. Analyseur (100) selon l'une des revendications 1 à 5,
pour lequel les cloisons (19.1, 19.2) tubulaires sont constituées d'un matériau au moins partiellement transparent pour le rayonnement thermique, notamment le rayonnement infrarouge.

7. Analyseur (100) selon l'une des revendications 1 à 6,
pour lequel le dispositif de chauffage (6) est disposé dans l'une des chambres annulaire (7.2) du système de conduite de gaz d'alimentation (7).

8. Analyseur (100) selon l'une des revendications 1 à 7,
pour lequel le dispositif de génération d'oxygène est relié avec la conduite d'alimentation en oxygène du réacteur digesteur (4) pour l'introduction de l'oxygène généré par le dispositif de génération d'oxygène dans le réacteur digesteur (4).

9. Analyseur (100) selon l'une des revendications 1 à 8,
pour lequel la membrane (9) est conçue en tant que tube membranaire fermé d'un côté, la membrane comprenant un côté rétentat extérieur faisant face à l'espace de réaction et un côté perméat intérieur faisant face à l'intérieur du tube membranaire.

10. Analyseur (100) selon la revendication 9,
pour lequel l'intérieur du tube membranaire est relié via une pompe (12) avec la conduite d'alimentation en oxygène du réacteur digesteur, et pour lequel la pompe (12) est conçue de telle sorte à générer, à l'intérieur du tube membranaire, une dépression par rapport à la pression régnant dans l'espace de réaction.

11. Analyseur (100) selon la revendication 9 ou 10,
pour lequel les diamètres et les longueurs des chambres annulaires (7.1, 7.2, 7.3) ainsi que le débit du gaz d'alimentation à travers le système de conduite de gaz d'alimentation (7) sont assortis à la longueur de la partie de tube membranaire disposée à l'intérieur de l'espace de réaction, de telle sorte que pendant le fonctionnement de l'analyseur, le tube membranaire présente, sur toute la longueur de la partie disposée à l'intérieur de l'espace de réaction, une température supérieure à une valeur seuil de température prédéfinie, qui correspond à la température de fonctionnement minimale de la membrane.

12. Analyseur (100) selon l'une des revendications 9 à 11,
pour lequel la largeur annulaire de la section de l'espace de réaction s'élève à 0,01 et 0,1 fois la longueur de la partie de tube membranaire se trouvant à l'intérieur de l'espace de réaction.
